# EUROPEAN PATENT APPLICATION

(11) **EP 3 275 366 A1**
(43) Date of publication of application: **31.01.2018**
(21) Application number: 16020280.0
(22) Date of filing: 27.07.2016
(51) Int. Cl.: A61B 5/05, G01R 33/02, A61B 8/15

(54) **REFRACTIVE ULTRASOUND SCANNER**

(71) Applicant: CERAGOS Electronics & Nature, 06800 Cagnes-sur-Mer (FR)
(72) Inventor: Cerasani, Luca, 06800 CAGNES-SUR-MER (FR)

(57) **Abstract**

Today, Ultrasounds Medical imaging is based on analysis of reflected Ultrasound waves across tissues. This methodology in spite of being largely implemented and offering several advantages such as non-invasiveness and safety presents a few drawbacks such as limited useful depth of analysis and image quality is deteriorated by artifacts linked to multiple reflections.

The new methodology will use refracted (penetrating) ultrasound waves. The volume to be analyzed is subjected to a strong but harmless Magnetic Field and then it is scanned by Focused Ultrasound waves. Magnetic Field generated by vibrating micro-volumes of tissues are detected by magnetic sensors like search coils or Anisotropic Magneto-Resistor (AMR).

The useful depth of analysis is significantly increased and refracted Ultrasound waves will "follow" paths with lower reflection coefficient e.g. blood vessels providing a complementary analysis to present systems. Image quality will not suffer of most reflection waves artifacts.

## Description

### Introduction :

Today, Ultrasounds Medical imaging is based on analysis of reflected Ultrasound waves across tissues. This methodology in spite of being largely implemented and offering several advantages such as non-invasiveness and safety presents the following drawbacks:
- Duplicated path to/from target tissue (incident and reflected Ultrasound waves)
- Inherent artifacts due to multiple waves reflections
- Use of Transducer with high Intensity dynamic range is required because the Transducer is also used for reception of Ultrasound waves

Four D systems (3D plus Time) have been developed as well as color imaging.

### Description :

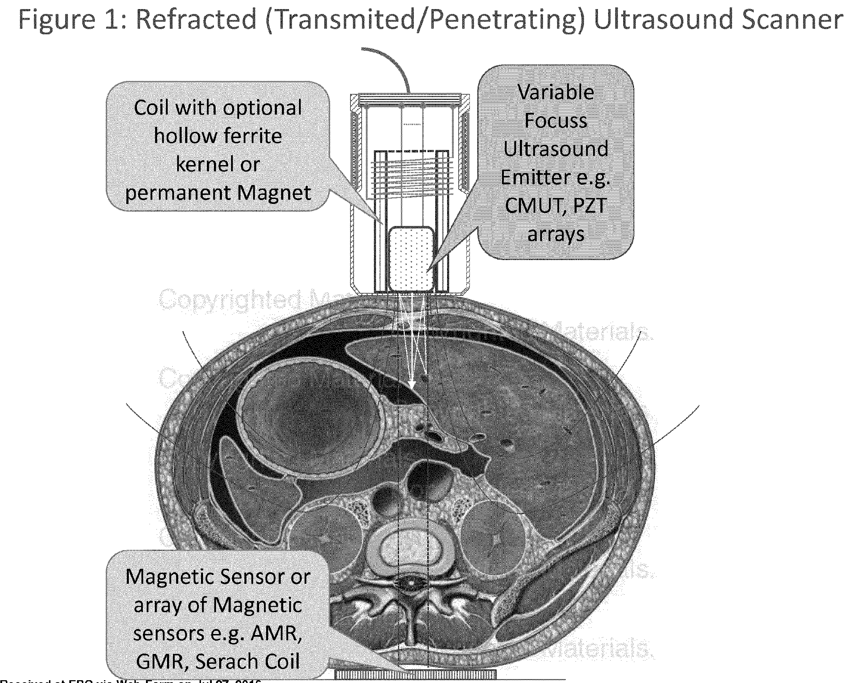

The system can be split into 4 main parts (Also see here joined Figure 1):
- A permanent Magnet or a Coil to slightly magnetize the tissues to be analyzed. Magnetic field generated can be either constant or variable according to time or according to position
- An Ultrasound Emitter generating Ultrasounds with variable intensity and frequency (typically less or equal to 1 MHz). The Ultrasound Emitter should be able to either focus the beam at any micro volume of the tissue to be analyzed or is made of an array of independent micro emitting devices such as CMUT (Capacitive Micro-machined Ultrasound Transducers)
- A magnetic sensor such as a search Coil or a Anisotropic Magneto-Resistor (AMR) is used to detect the variation of the magnetic field in the tissue micro volume when the Ultrasound beam is focused on it. The sensor can be replaced by an array of micro magnetic sensors to allow the detection of the position of the Ultrasound beam (on the plane perpendicular to the beam direction) especially when beam is not focused and Ultrasound waves are guided by tissues or liquids with lowest absorption coefficient such as Ultrasounds waves in blood inside vessels
- An Electronic circuit used to control all elements and provide a visual output

### Notes:

- As attenuation of Ultrasound waves is proportional to the logarithm of the frequency it is possible to evaluate the attenuation of the refracted waves at any time by slightly modifying the Emitter frequency considering that the amplitude of detected signal is proportional to the frequency and to the exponential of the attenuation coefficient which is also dependent of the frequency (starting from first tissue micro layer and one micro layer by one micro layer)
- If the speed of Ultrasound waves when crossing various tissues cannot be considered as constant the temporal position of the wave (in the wave direction) cannot be evaluated by registration of the phase variation of sensed magnetic field variations due to vibration of contiguous micro-tissue volumes because it is independent of the speed of Ultrasounds. The focus point distance (also independent of speed of Ultrasounds) can be used as an evaluation of the depth of the analyzed micro-volume of tissue or a sensors array aligned with the Ultrasound wave direction could alternatively be implemented.

## Claims

1. A new method to analyze and visualize internal tissues by using refracted waves instead of echoed/reflected waves.
Refracted Ultrasound waves can be monitored using Magnetic sensors provided tissues to be analyzed are slightly magnetized using a permanent magnet or a coil with a ferrite kernel.
Compared to current technology it offers the following advantages:
• One way Ultrasound waves providing an increased depth of tissue analysis
• Significant decrease of artifacts linked to Reflected waves is expected because only transmitted waves are monitored and direction of Ultrasound waves are better set as perpendicular to the tissues surface
• Ultrasound waves will "follow" tissues with lower reflection coefficient like Blood in vessels providing a complementary analysis to present systems
• Less complex Ultrasound transducer circuitry can be envisaged

## Amended claims

### Amended claims in accordance with Rule 137(2) EPC.

1. A new method to analyze and visualize internal tissues by using refracted Ultrasound waves instead of echoed/reflected waves.
Electromagnetic detection is typically used but not exclusively and Microwave detection is not needed.
This methodology is non-invasive and does not require the analyzed tissues to be charged.
